# EUROPEAN PATENT SPECIFICATION

(11) **EP 3 004 173 B1**
(45) Date of publication and mention of the grant of the patent: **30.05.2018**
(21) Application number: 14733677.0
(22) Date of filing: 05.06.2014
(51) Int. Cl.: C07K 16/46, C07K 14/005, C12N 15/62, A61K 39/00, G01N 33/50

(54) **SINGLE DOMAIN ANTIBODY DISPLAY**
DISPLAY VON EINZELDOMÄNEN-ANTIKÖRPER
PRÉSENTATION DES ANTICORPS À DOMAINE UNIQUE

(30) Priority: 06.06.2013 US 201361831707 P
(43) Date of publication of application: 13.04.2016
(73) Proprietor: iQur Limited, London NW1 0NH (GB)
(72) Inventor: Rowlands, David J., Leeds LS2 9JT (GB); Lomonossoff, George, Norwich, NR4 7UH (GB); Peyret, Hadrien, Norwich, NR4 7UH (GB)
(74) Representative: J A Kemp
(86) International application number: PCT/GB2014/051739
(87) International publication number: WO 2014/195713

(56) References cited:
- WO-A1-01/77158
- WO-A2-2011/116226
- GOYVAERTS C ET AL: "Development of the Nanobody display technology to target lentiviral vectors to antigen-presenting cells", GENE THERAPY, vol. 19, no. 12, December 2012 (2012-12), pages 1133-1140, XP002729509,

## Description

### FIELD OF THE INVENTION

The invention relates to fusion proteins comprising a single-domain antibody, nucleic acid molecules encoding the proteins, processes for producing the proteins, pharmaceutical and vaccine compositions containing the proteins and use of the proteins in therapy, vaccination and diagnosis.

### BACKGROUND OF THE INVENTION

Antibodies are important tools for experimental research and medical applications. Most antibodies are composed of two heavy and two light chains, both of which contribute to the two identical antigen-binding sites. In addition to these conventional antibodies, camelids and some cartilaginous fish such as sharks also produce antibodies composed only of heavy chains. The antigen-binding site of these heavy chain antibodies (hcAbs) is formed only by a single domain, designated VHH (Variable domain of the Heavy chain of the Heavy-chain antibody) in camelid hcAbs and VNAR (Variable domain of the shark New Antigen Receptor) in cartilaginous fish hcAbs. VHH and VNAR can be produced as recombinant proteins, designated single-domain antibodies (sdAbs).

### BRIEF SUMMARY OF THE INVENTION

The invention is concerned with a highly flexible antibody presentation system based on tandem hepatitis B (HBV) core proteins. The inventors have demonstrated that it is possible to present a single-domain antibody (sdAb) in an active (i.e antigen-binding) form on the surface of HBV core antigen (HBcAg) particles by using tandem HBV core constructs. This type of antibody display has multiple uses, including use in therapy, vaccination and diagnostic uses.

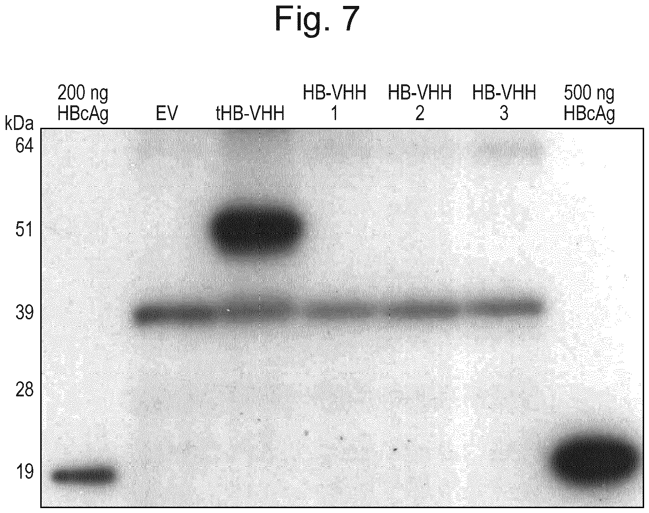

Interestingly, antibody display on the surface of tandem core proteins in plants was attempted with the more commonly used single-chain variable fragment antibodies (scFv). However, expression yields were very low and no particle formation was observed. This is most likely due to the more complicated structure of scFv, which have two domains corresponding to the variable regions of the light and heavy chains, whereas sdAb only have a single domain corresponding to the variable region of the heavy chain. It is also interesting to note that an sdAb (VHH) could not be displayed on the surface of monomeric (i.e. non-tandem) HBcAg particles in plants: the recombinant protein could not be detected (Figure 7), indicating that folding problems or steric hindrance issues probably caused immediate degradation on the protein.

Thus, the invention provides a protein comprising a first and a second copy of hepatitis B core antigen (HBcAg) in tandem, in which one or both of the copies of HBcAg comprises a sdAb in the e1 loop.

The flexibility of the technology means that one or both of the copies of HBcAg may comprise a sdAb in the e1 loop. Essentially, as long as one of the copies of HBcAg comprises a sdAb in the e1 loop, the second copy of HBcAg may comprise any protein of interest. Thus, the second copy of HBcAg may also comprise a sdAb in the e1 loop that is the same as or different from the sdAb in the e1 loop of the first copy of HBcAg. Alternatively, the second copy of HBcAg may comprise a different heterologous protein in the e1 loop.

The antibody display technology has multiple uses, some of which may require antigen to be loaded onto the sdAb that is presented on the tandem core protein and some of which do not.

For example, the tandem core protein may be engineered to present a therapeutic antibody for use in the treatment of a disease or condition of the human or animal body. An advantage of using the tandem core protein to display the therapeutic antibody is that multiple copies of the protein can be present in a Virus-Like Particle (VLP) or Core-Like Particle (CLP), enabling a high dose of the therapeutic antibody to be administered. The technique greatly increases the biological activity per unit since each particle contains 90-120 copies of the antibody. In addition, the flexibility of the system as mentioned above means that more than one type of sdAb could be administered simultaneously in a precise 1:1 ratio.

As another example, the tandem core protein may be engineered to present a sdAb that can be loaded with antigen for use as a vaccine for presenting the antigen to the mammalian immune system. In other words, an antigen can be displayed on the VLP or CLP surface and held there by an antibody-antigen interaction. Because antigens are more immunogenic when presented on the surface of a tandem core, such a system may be suitable for the display of antigens that have low inherent immunogenicity. This technology may be particularly suited to antigens that need to be presented in a particular conformation to be recognised by the immue system.

The protein of the invention can also be used for diagnostic purposes. For example, the tandem core protein may be engineered to present a sdAb that is capable of binding to an antigen of interest that can be used to detect the presence of the antigen. The second copy of HBcAg may comprise a means of visualising the protein. For example, the second copy of HBcAg could comprise a fluorescent or bioluminescent protein in the e1 loop. It has previously been shown that the large Green Fluorescent Protein (GFP) can be carried by tandem core. Therefore, if a single chain antibody was carried alongside it in the second core then a simple fluorescent assay for any analyte could be designed. For example, the target analyte could be bound to an ELISA plate and then the fluorescent VLP added. A simple measure of fluorescence would determine the presence of target. Whilst this initially appears similar to a conventional ELISA, it has the added advantage of massively increased sensitivity due to the multiple copies of the antibody present. As a comparator, streptavidin-biotin increases sensitivity of ELISAs 4-fold; the present invention could increase this x100 fold.

The invention also provides:
- a particle comprising multiple copies of a protein of the invention;
- a nucleic acid molecule encoding a protein of the invention;
- a host cell comprising a nucleic acid molecule of the invention;
- a process for producing a protein of the invention, which process comprises culturing a host cell containing a nucleic acid molecule which encodes the protein under conditions in which the protein is expressed, and recovering the protein;
- a pharmaceutical composition comprising a protein of the invention, a particle of the invention or a nucleic acid molecule of the invention and a pharmaceutically acceptable carrier or diluent;
- a vaccine comprising a protein of the invention, a particle of the invention or a nucleic acid molecule of the invention and a pharmaceutically acceptable carrier or diluent;
- a protein of the invention or a particle of the invention for use in a method of treatment of the human or animal body;
- a protein of the invention or a particle of the invention for use in a method of vaccination of the human or animal body;
- use of a protein of the invention or a particle comprising multiple copies of a protein of the invention, for detecting an antigen in a sample; and
- a method of detecting an antigen in a sample, comprising applying a protein of the invention or a particle comprising multiple copies of a protein of the invention to the sample.

### BRIEF DESCRIPTION OF THE DRAWINGS

Figure 1: Diagram of possible tandem core construct. A sdAb may be inserted into Insert I and/or Insert II.
Figure 2: (A) X-ray crystallographic data shows the natural dimer formed by HBV core protein. This is the main building block of the resulting virus like particle. (B) Molecular modelling suggests that the folding of tandem-core constructs appears to be indistinguishable from monomeric cores. Note the addition of a linker sequence between the two cores ensuring that they are expressed as a single protein.
Figure 3: (A) Electron microscopy of VLPs formed by monomeric (left) and tandem (right) core proteins. VLPs appear to have an identical morphology. (B) Cryo-electron microscopy confirms that monomeric (left) and tandem (right) core constructs fold in an identical manner.
Figure 4: Electron micrographs of tHB-VHH core-like particles. Top: particles without GFP (scale bar 20 nm); bottom: particles with GFP (scale bar, 100 nm).
Figure 5: Different plant extracts run on sucrose cushions. After ultracentrifugation, GFP only runs down to the high-concentration sucrose when in the presence of tHB-VHH (particles which display an anti-GFP antibody fragment). The results demonstrate that the tHB-VHH particles bind GFP, indicating that the VHH is properly folded and active on the surface of the core-like particles (CLPs).
Figure 6: Western blots of sucrose gradient fractions. Top membrane: blot with anti-GFP antibody; bottom membrane: blot with anti-HBcAg antibody. S: supernatant clarified extract, Top: top fraction of the sucrose cushion, Mid: middle fraction, Bott: bottom fraction. GFP largely remains in the supernatant of the gradients when it is on its own or with tHB, but in the presence of tHB-VHH, it is dragged to the bottom of the cushion and co-localises with the antibody-bearing particles.
Figure 7: Western blot of crude plant extracts with anti-HBcAg antibody. EV: empty vector control - plants agroinfiltrated with pEAQ-HT vector containing no heterologous sequence in the T-DNA multiple cloning site. tHB-VHH: tandem HBcAg construct with anti-GFP VHH in C-terminal E1 loop. HB-VHH: monomeric HBcAg containing anti-GFP VHH in e1 loop; numbers 1-3 refer to the individual *Agrobacterium* transformant colony from which the inoculums originated (three separate clones were tested). The 39 kDa band is in all samples including the empty vector control, indicating that it reflects non-specific signal.

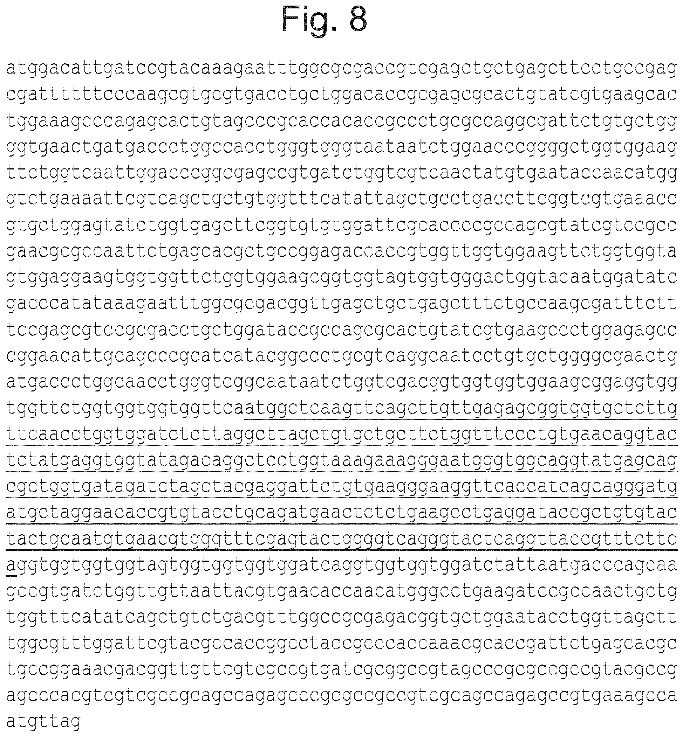

Figure 8: Sequence encoding the tHBcAg-anti-GFP construct described in the Example below. The sequence encoding the VhH part of the construct is underlined.
Figure 9: Amino acid sequence of the tHBcAg-anti-GFP construct described in the Example below. The sequence of the VhH part of the construct is underlined. The linker between the tandem cores is shown in bold and italics.
Figure 10: Amino acid sequence of the anti-GFP VhH used in the experiments described in the Example below.

### BRIEF DESCRIPTION OF THE SEQUENCES

SEQ ID NO: 1 is the 183 amino acid protein of the ayw subtype plus a 29 amino acid pre-sequence of HBcAg and the corresponding nucleotide sequence.
SEQ ID NO: 2 is the 183 amino acid protein of the ayw subtype plus a 29 amino acid pre-sequence of HBcAg.

### DETAILED DESCRIPTION OF THE INVENTION

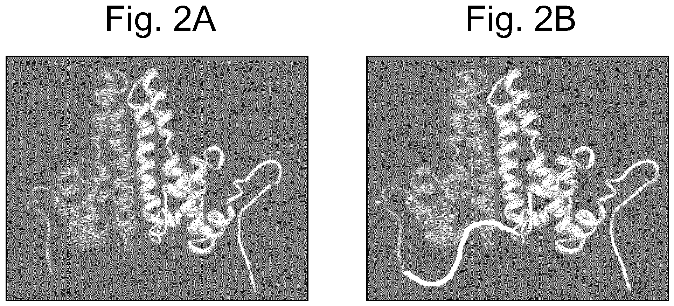

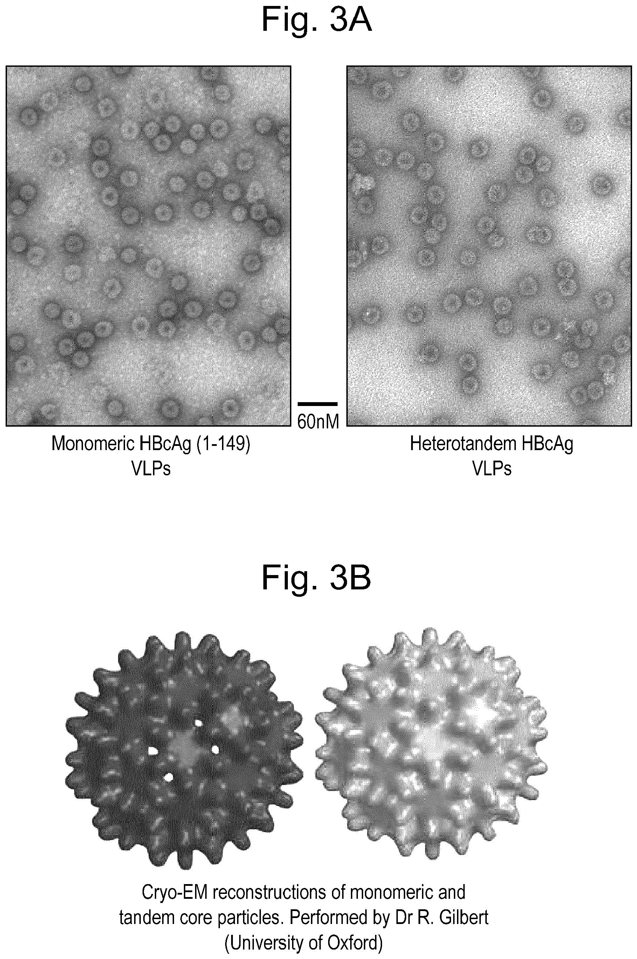

Tandem core constructs are a genetic fusion of two hepatitis B (HBV) core genes such that the resulting recombinant protein forms two parallel "spikes" which are indistinguishable from wild type core proteins which naturally dimerise (Figure 2). Tandem core proteins form virus like particles (VLPs) in a manner similar to monomeric HBV core protein (Figure 3).

The inventors have demonstrated that it is possible to present a sdAb in an active form on the surface of HBV core antigen (HBcAg) particles by using tandem HBV core constructs.

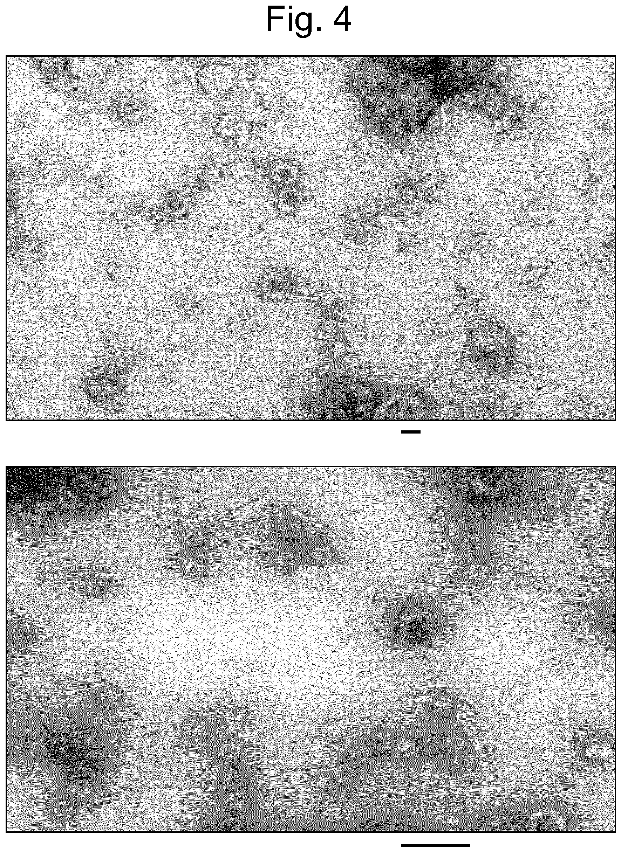

In more detail, the inventors designed a tandem core construct comprising a sdAb (VHH) in the e1 loop of one of the copies of HBcAG. The VHH was an anti-GFP VHH, isolated from an immunized alpaca-derived VHH phage display library. The gene coding for this VHH was cloned into the C-terminal monomer of the tandem core construct. This construct (tHB-VHH) was expressed transiently in *Nicotiana benthamiana* and directed the production of core-like particles (CLPs) displaying the VHH on the surface (Figure 4).

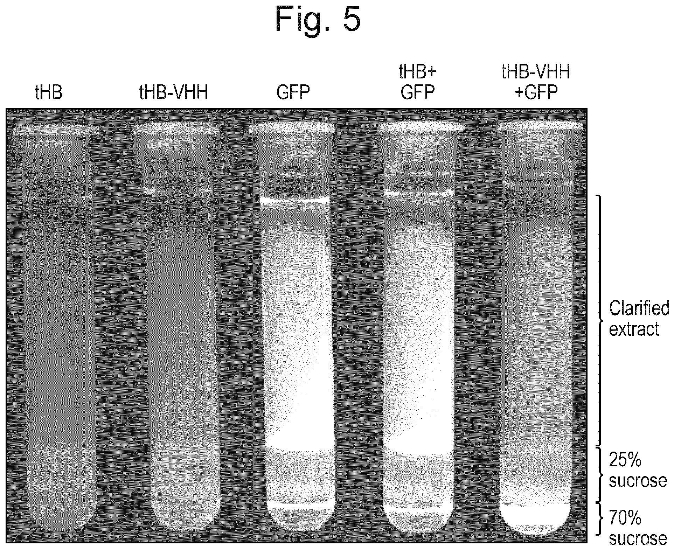

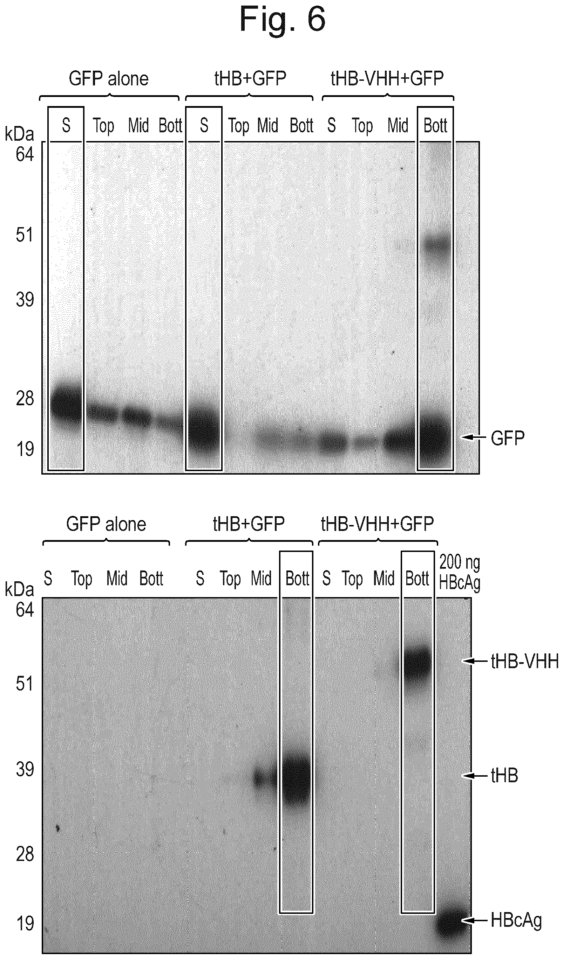

The particles were shown to cause GFP to migrate down a sucrose gradient, which it does not normally do as it is not dense enough on its own or in the presence of non-VHH bearing tHB particles (Figure 5). This clearly shows that the tHB-VHH particles bind GFP, thus indicating that the VHH is properly folded and active on the surface of the CLPs. Western blot analysis of the sucrose cushion fractions confirmed that GFP remains largely in the supernatant when run on its own or with tHB, but when run with tHB-VHH, GFP and CLPs co-localise at the bottom of the cushion (Figure 6).

Thus, the inventors have, for the first time, demonstrated that it is possible to present a VHH on the surface of HBcAg particles by using tandem HBV core constructs. The experiments showed that the VHH was correctly folded and active.

Initial experiments were aimed at displaying a scFv antibody in the e1 loop of HBcAg. However this proved problematic. Expression yields were very low and no particle formation was observed (data not shown). This is most likely due to the more complicated structure of scFv, which have two domains corresponding to the variable regions of the light and heavy chains, whereas VHH only have a single domain corresponding to the variable region of the heavy chain. This increased complexity of scFv probably prevented proper folding of the insert when both N- and C-termini were attached to the e1 loop of HBcAg, leading to improper folding of the entire tandem molecule.

Furthermore, VHH could not be displayed on the surface of monomeric (i.e. non-tandem) HBcAg particles in plants: the recombinant protein could not be detected (Figure 7), indicating that folding problems or steric hindrance issues probably caused immediate degradation on the protein.

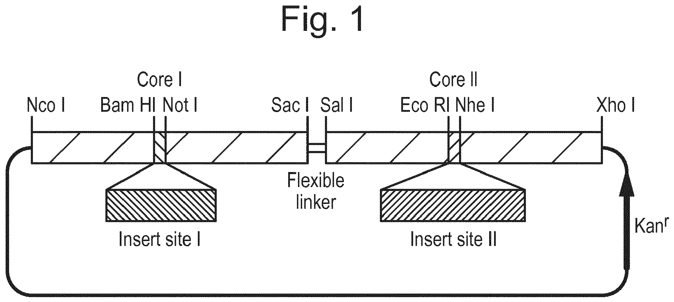

The invention is therefore concerned with a novel antibody-presentation system based on tandem HBV core proteins. In particular, the invention provides a protein comprising a first and a second copy of HBcAg in tandem, in which one or both of the copies of HBcAg comprises a sdAb in the e1 loop. Figure 1 shows an example tandem core construct in which a nucleic acid encoding an sdAb may be inserted into one or both insert sites.

### The Core Protein

The basic building block of the protein of the invention is HBcAg, which has 183 or 185 amino acids (aa) depending on the subtype of HBV. The sequence of the 183 amino acid protein of the ayw subtype plus a 29 amino acid pre-sequence is shown in SEQ ID NO: 2. The mature HBcAg runs from the Met residue at position 30 to the Cys residue at the extreme C-terminus, with the sequence from positions 1 to 29 being a pre-sequence.

The protein generally comprises only two copies of HBcAg forming a dimer because dimers of HBcAg form the structural building blocks of core particles. The HBcAg units are generally joined together in a head-to-toe fashion, i.e. the C-terminus of one unit is joined to the N-terminus of the adjacent unit. The units may be joined directly by a covalent bond (e.g. a peptide bond), but preferably they are joined by a linker which spaces the adjacent units apart and thereby prevents any problem with disruption of the packing of adjacent units. The nature of the linker is discussed below.

One or both of the HBcAg units in the protein of the invention may be native full length HBcAg. However, at least one of the units is a modified form of HBcAg having a sdAb inserted into the e1 loop. Both of the HBcAg units may have a sdAb inserted into the e1 loop. When only one of the HBcAg units has a sdAb inserted into the e1 loop, the other unit may be native HBcAg or it may have a heterologous protein inserted into the e1 loop. Examples of possible heterologous proteins are discussed below.

As a general rule, any modifications are chosen so as not to interfere with the conformation of HBcAg and its ability to assemble into particles. Such modifications are made at sites in the protein which are not important for maintenance of its conformation, for example in the e1 loop, the C-terminus and/or the N-terminus. The e1 loop of HBcAg can tolerate insertions of e.g. from 1 to 120 amino acids without destroying the particle-forming ability of the protein.

The HBcAg sequence may be modified by a substitution, insertion, deletion or extension. The size of insertion, deletion or extension may, for example, be from 1 to 200 aa, from 3 to 100 aa or from 6 to 50 aa. Substitutions may involve a number of amino acids up to, for example, 1, 2, 5, 10, 20 or 50 amino acids over the length of the HBcAg sequence. An extension may be at the N- or C-terminus of HBcAg. A deletion may be at the N-terminus, C-terminus or at an internal site of the protein. Substitutions may be made at any position in the protein sequence. Insertions may also be made at any point in the protein sequence, but are typically made in surface-exposed regions of the protein such as the e1 loop. More than one modification may be made to each HBcAg unit. Thus, it is possible to make a terminal extension or deletion and also an internal insertion. For example, a truncation may be made at the C-terminus and an insertion may be made in the e1 loop.

Each part of the HBcAg sequence in the protein of the invention preferably has at least 70% sequence identity to the corresponding sequence of a natural HBcAg protein, such as the protein having the sequence shown in SEQ ID NO: 2. More preferably, the identity is at least 80%, at least 90%, at least 98%, at least 97% or at least 99%. Methods of measuring protein homology are well known in the art and it will be understood by those of skill in the art that in the present context, homology is calculated on the basis of amino acid identity (sometimes referred to as "hard homology").

For example the UWGCG Package (Devereux et al (1984) Nucleic Acids Research 12: 387-395) provides the BESTFIT program which can be used to calculate homology (for example used on its default settings). The PILEUP and BLAST algorithms can be used to calculate homology or line up sequences (typically on their default settings), for example as described in Altschul S. F. (1993) J Mol Evol 36:290-300; Altschul, S, F et al (1990) J Mol Biol 215:403-10.

Software for performing BLAST analyses is publicly available through the National Center for Biotechnology Information (http://www.ncbi.nlm.nih.gov/). This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul et al, supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) Proc. Natl. Acad. Sci. USA 89: 10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) Proc. Natl. Acad. Sci. USA 90: 5873-5787. One measure of similarity provided by the BLAST algorithm is the smallest sum probability (P(N)), which provides an indication of the probability by which a match between two nucleotide or amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

The e1 loop of HBcAg is at positions 68 to 90, and a heterologous protein, such as a sdAb or a different heterologous protein, may be inserted anywhere between these positions. Preferably, the protein is inserted in the region from positions 69 to 90, 71 to 90 or 75 to 85. Most preferred is to insert the protein between amino acid residues 79 and 80 or between residues 80 and 81. When a heterologous protein is inserted, the entire sequence of HBcAg may be maintained, or alternatively the whole or a part of the e1 loop sequence may be deleted and replaced by the heterologous sequence. Thus, amino acid residues 69 to 90, 71 to 90 or 75 to 85 may be replaced by a heterologous protein. Where a heterologous protein replaces the e1 loop sequence, the protein is generally not shorter than the sequence that it replaces.

A C-terminal truncation of HBcAg will generally not go beyond aa 144 because if any further truncation is made particles may not form. Thus, the deleted amino acids may, for example, comprise aa 144 to the C-terminal aa (aa 183 or 185), aa 150 to the C-terminal aa, aa 164 to the C-terminal aa or aa 172 to the C-terminal aa. The C-terminus of HBcAg binds DNA, and truncation of the C-terminus therefore reduces or completely removes DNA from preparations of HBcAg and HBcAg hybrid proteins.

The protein of the invention forms particles which preferably resemble the particles formed by native HBcAg. The particles of the invention are typically at least 10 nm in diameter, for example from 10 to 50 nm or from 20 to 40 nm in diameter, but preferably they are about 27 nm in diameter (which is the size of native HBcAg particles). They comprise multiple HBcAg units, for example from 150 to 300 units, but generally they are fixed to about 180 or about 240 units (which are the numbers of units in native HBcAg particles). As the protein of the invention is a dimer, this means that the number of protein monomers in the particles may be from 75 to 150 but is generally about 90 or about 120.

The linker between adjacent HBcAg units and/or flanking the inserts in e1 loop is generally a chain of amino acids at least 1.5 nm (15 Å) in length, for example from 1.5 to 10 nm, from 1.5 to 5 nm or from 1.5 to 3 nm. It may, for example, comprise 4 to 40 aa or 10 to 30 aa, preferably 15 to 21 aa. The linker is generally flexible. The amino acids in the linker may, for example, include or be entirely composed of glycine, serine and/or proline. For example, the linker may comprise one or more repeats of the sequence GlyₙSer(GₙS) wherein n is from 2 to 8. A preferred linker comprises one or more repeats of the sequence GlyGlyGlyGlySer (GGGGS). Alternatively, the linker may comprise one or more GlyPro (GP) dipeptide repeats. The number of repeats may, for example, be from 1 to 18, preferably from 3 to 12. In the case of GGS repeats, the use of 5, 6 or 7 repeats has been found to allow the formation of particles. The linker may correspond to the hinge region of an antibody; this hinge region is thought to provide a flexible joint between the antigen-binding and tail domains of antibodies.

### Single-domain antibody (sdAb)

The protein of the invention presents at least one sdAb and has multiple potential uses, for example in therapeutic, vaccination and/or diagnostic methods. The protein of the invention has a sdAb inserted into the e1 loop of one or both copies of HBcAg. The protein of the invention can have a sdAb inserted into the e1 loop of both copies of HBcAg.

SdAb are antibody fragments derived from heavy-chain antibodies of camelids and cartilaginous fish. The antigen-binding site of heavy-chain antibodies is confined to one single domain referred to as the VHH in Camelidae and VNAR in cartilaginous fish. These variable domains can be easily expressed in bacteria, yeasts or in other hosts as recombinant single-domain antibodies. Like conventional antibodies, sdAb can have high target specificity and high affinity for their target. However, as a result of their small size, sdAb can recognise uncommon epitopes on targets hidden or shielded from the much large conventional antibodies. SdAb and their applications are reviewed in detail in Eyer and Hruska, 2012.

Any sdAb can be used in the invention. An sdAb is the variable domain of a heavy polypeptide chain of an immunoglobulin devoid of light chains. The sdAb may be derived from a heavy-chain antibody from Camelidae or cartilaginous fish.

The sdAb may be specific to, or raised against, a particular antigen. The choice of sdAb and/or the antigen it binds to will of course depend on the intended use of the protein of the invention. For example, if the intended use is a therapy, the sdAb will be a therapeutic antibody. The protein of the invention may comprise an sdAb that is useful in the treatment of any disease or condition.

If the protein of the invention is intended for use as a vaccine, the sdAb to be used will depend on the disease that it is wished to vaccinate against. The sdAb may, for example, bind to an antigen from a pathogenic organism, to a cancer-associated antigen or an allergen. The pathogenic organism may, for example, be a virus, a bacterium or a protozoan.

The sdAb will have the ability to bind to an antigen of interest. Preferably, the sdAb will bind specifically to the antigen of interest. That is, the sdAb will preferably bind to the antigen of interest with greater binding affinity than that at which it binds to another molecule.

The term "binding affinity" is intended to refer to the tendency of an antibody to bind or not to bind to a target. Binding affinity may be quantified by determining the dissociation constant (Kd) for an antibody and its target. Similarly, the specificity of binding of an antibody to its target may be defined in terms of the comparative dissociation constants (Kd) of the antibody for its target as compared to the dissociation constant with respect to the antibody and another, non-target molecule.

Typically, the Kd for the antibody with respect to the target will be 2-fold, preferably 5-fold, more preferably 10-fold less than Kd with respect to the other, non-target molecule such as unrelated material or accompanying material in the environment. More preferably, the Kd will be 50- fold less, even more preferably 100-fold less, and yet more preferably 200-fold less. The value of this dissociation constant can be determined directly by well-known methods.

The sequence of the sdAb for cloning into the tandem core construct may be determined or designed by any method known in the art. For example, sdAbs may be generated by PCR cloning of the variable domain repertoire from blood, lymph node, or spleen cDNA obtained from immunised animals, such as a camels or llamas, into a phage or ribosome display vector. Antigen-specific sdAbs may be selected by panning phage or ribosome libraries on immobilised antigen, e.g. antigen coated onto the plastic surface of a test tube, biotinylated antigens immobilized on Streptavidin beads, or membrane proteins expressed on the surface of cells. It is also possible to construct semi-synthetic libraries by cassette-mutagenesis of the Complementarity Determining Regions (CDRs). This offers the advantage of selecting antibodiesagainst toxic or difficult to express antigens. In addition, the affinity of sdAbs for the desired antigen can be improved by site directed mutagenesis of the CDRs and further rounds of panning on immobilized antigen under conditions of increased stringency (higher temperature, high or low salt concentration, high or low pH, and low antigen concentrations).

If the protein of the invention is for administration to a human subject, the sdAb is preferably humanised. Methods for humanising sdAb are known in the art.

A full length sdAb may be inserted into the e1 loop of HBcAg. The sdAb may have the sequence of a naturally occurring sdAb or may be a variant of a naturally occurring sdAb sequence.

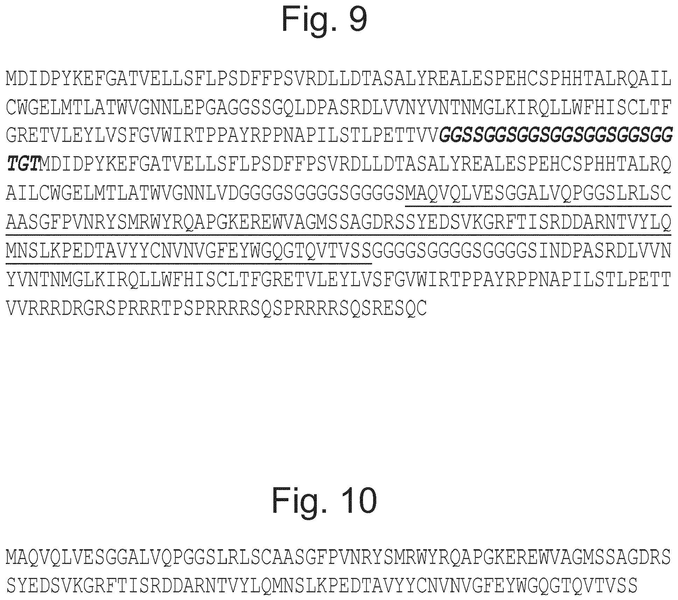

The sequence of the sdAb may have homology with the sequence shown in Figure 10 such as at least 60% identity, at least 80%, at least 90%, at least 95%, at least 97% or at least 99% identity, for example over the full sequence or over a region of at least 20, preferably at least 30, for instance at least 40, at least 50, at least 60, or at least 80 or more contiguous amino acids. Methods of measuring protein homology are well known in the art and are discussed above in relation to the HBV core protein.

The sdAb to be inserted into the protein of the invention may be of any suitable size that does not disrupt VLP formation, such as 5 to 20 kDa. sdAbs typically have molecular weights in the range of 12 to 15 kDa.

The protein of the invention may comprise two sdAb, for example by comprising one sdAb in each copy of HBcAg. This may improve the affinity of the protein of the invention for one or more antigens. The two sdAb may be the same or different. If the two sdAb are different, they may bind to the same or different antigens.

### Other Heterologous Proteins or Epitopes

The flexibility of the tandem core system means that the protein of the invention, in addition to comprising a sdAb, may comprise one or more further heterologous proteins or epitopes. The one or more heterologous proteins or epitopes may be in the first or second copy of HBcAg. Preferably, the one or more heterologous proteins or epitopes are in the copy of HBcAg that does not comprise the sdAb.

For example, the protein of the invention may comprise one or more heterologous epitopes in order to induce an immune response to the one or more heterologous epitopes. Thus, it would be possible to create a vaccine comprising the protein of the invention in which a sdAb is bound to an antigen for use in inducing an immune response in a subject to the antigen. In addition, the protein could comprise one or more heterologous epitopes that would also induce an immune response. The heterologous epitopes may relate to the same or a different antigen to the one that is bound to the sdAb.

As another example, the protein of the invention may comprise a heterologous protein that acts as a means of visualising the protein of the invention. This might be useful in a diagnostic method. Examples of such a heterologous protein are fluorescent or bioluminescent proteins such as Green Fluorescent Protein (GFP).

Therefore, although the protein of the invention must have a sdAb inserted into the e1 loop of at least one copy of HBcAg, the e1 loop of the other copy of HBcAg in the protein may comprise any other type of heterologous protein or epitope.

A "heterologous" protein or epitope is a protein or epitope that is not normally located at the position at which it is located in the HBcAg; it is generally from a protein other than HBcAg but it may be from a different location in HBcAg. For example, it may be HBV surface antigen (sAg). If the protein of the invention comprises a heterologous epitope, it comprises a sequence of amino acids which raises an immune response. The epitope may be conformational or linear. It may be, for example, in a sequence of from 6 to 120 aa, from 6 to 50 aa or from 6 to 20 aa. A major advantage of the invention is that it allows epitopes carried on large sequences to be inserted into the e1 loop, for example on sequences of from 30 to 120 aa, 40 to 120 aa or 60 to 120 aa.

The heterologous protein or epitope to be used as an insert may be of any suitable size that does not disrupt VLP formation. It is preferably less than 40 kDa, less than 20 kDa, less than 15kDa, less than 10 kDa or less than 5 kDa.

The protein of the invention may contain more than one heterologous protein or epitope, for example up to 2, 3, 5 or 8 heterologous proteins or epitopes. More than one copy of a protein or epitope may be inserted in a HBcAg unit; for example, from 2 to 8 copies may be inserted. Where there are two or more heterologous proteins or epitopes in the protein of the invention, they may be from the same or different organisms.

The epitope may be a T-cell or a B-cell epitope. If it is a T-cell epitope, it may be a cytotoxic T-lymphocyte (CTL) epitope or a T-helper (Th) cell epitope (e.g. a Th1 or Th2 epitope). In a preferred embodiment of the invention, one of the epitopes is a T-helper cell epitope and another is a B-cell or a CTL epitope. The presence of the T-helper cell epitope enhances the immune response against the B-cell or CTL epitope.

The choice of epitope depends on the disease that it is wished to vaccinate against. The epitope may, for example, be from a pathogenic organism, a cancer-associated antigen or an allergen. The pathogenic organism may, for example, be a virus, a bacterium or a protozoan.

### Making the proteins of the invention

The proteins of the invention are generally made by recombinant DNA technology. The invention includes a nucleic acid molecule (e.g. DNA or RNA) encoding a protein of the invention, such as an expression vector. The nucleic acid molecules may be made using known techniques for manipulating nucleic acids (or may simply be bought). Typically, two separate DNA constructs encoding two HBcAg units are made and then joined together by overlapping PCR.

A protein of the invention may be produced by culturing a host cell containing a nucleic molecule encoding the protein under conditions in which the protein is expressed, and recovering the protein. Suitable host cells include bacteria such as *E. coli,* yeast such as *Pichia pastoris,* mammalian cells and other eukaryotic cells, for example insect Sf9 cells. The nucleic acid molecule could be expressed in a plant, such as *N. benthamiana.*

The vectors constituting nucleic acid molecules according to the invention may be, for example, plasmid or virus vectors. They may contain an origin of replication, a promoter for the expression of the sequence encoding the protein, a regulator of the promoter such as an enhancer, a transcription stop signal, a translation start signal and/or a translation stop signal. The vectors may also contain one or more selectable marker genes, for example an ampicillin resistance gene in the case of a bacterial plasmid or a neomycin resistance gene in the case of a mammalian vector. Vectors may be used *in vitro,* for example for the production of RNA or used to transform or transfect a host cell. The vector may also be adapted to be used *in vivo,* for example in a method of gene therapy or DNA vaccination.

Promoters, enhancers and other expression regulation signals may be selected to be compatible with the host cell for which the expression vector is designed. For example, prokaryotic promoters may be used, in particular those suitable for use in *E. coli* strains (such as *E. coli* HB101). A promoter whose activity is induced in response to a change in the surrounding environment, such as anaerobic conditions, may be used. Preferably an *htrA* or *nirB* promoter may be used. These promoters may be used in particular to express the protein in an attenuated bacterium, for example for use as a vaccine. When expression of the protein of the invention is carried out in mammalian cells, either *in vitro* or *in vivo,* mammalian promoters may be used. Tissue-specific promoters, for example hepatocyte cell-specific promoters, may also be used. Viral promoters may also be used, for example the Moloney murine leukaemia virus long terminal repeat (MMLV LTR), the rous sarcoma virus (RSV) LTR promoter, the SV40 promoter, the human cytomegalovirus (CMV) IE promoter, herpes simplex virus promoters and adenovirus promoters. All these promoters are readily available in the art.

A protein according to the invention may be purified using conventional techniques for purifying proteins. The protein may, for example, be provided in purified, pure or isolated form. For use in a vaccine, the protein must generally be provided at a high level of purity, for example at a level at which it constitutes more than 80%, more than 90%, more than 95% or more than 98% of the protein in the preparation. However, it may be desirable to mix the protein with other proteins in the final vaccine formulation.

### Use in therapy or in vaccination against diseases

The protein of the invention may be used in therapy or as a vaccine. The invention includes a pharmaceutical composition or a vaccine composition comprising a protein of the invention, a particle comprising multiple copies of the protein of the invention or a nucleic acid molecule encoding the protein of the invention and a pharmaceutically acceptable carrier or diluent. If the protein of the invention comprises a therapeutic sdAb, the composition can be used for the treatment of a human or animal body by therapy. If the protein of the invention comprises a sdAb that binds to an antigen that is capable of inducing an immune response in a subject, the composition can be used as a vaccine. A vaccine composition preferably comprises the antigen that binds to the sdAb.

The principle behind vaccination is to induce an immune response in a host so as to generate an immunological memory in the host. This means that, when the host is exposed to the virulent pathogen, it mounts an effective (protective) immune response, i.e. an immune response which inactivates and/or kills the pathogen. The invention forms the basis of a vaccine against any antigen of interest, such as a pathogen, a cancer-associated antigen or an allergen. The sdAb and any other heterologous proteins or epitopes in the protein of the invention are chosen so as to be appropriate for the disease or condition against which the vaccine is intended to provide protection.

A method of inducing an immune response in a subject is described, comprising administering to the subject the protein, particle or nucleic acid of the invention. Preferably, the antigen that binds to the sdAb is bound to the sdAb when the protein of the invention is administered. Therefore, to administer the protein of the invention as a vaccine, the protein presenting the sdAb of the invention is allowed to bind the antigen of interest before it is administered to the subject. SdAb bind to antigens with high affinity. However, it would be possible to additionally tether the antigen to the sdAb, for example using chemical conjugation such as an engineered disulphide bond.

The terms "individual" and "subject" are used interchangeably herein to refer to any member of the subphylum cordata, including, without limitation, humans and other primates, including non-human primates such as chimpanzees and other apes and monkey species; farm animals such as cattle, sheep, pigs, goats and horses; domestic mammals such as dogs and cats; laboratory animals including rodents such as mice, rats and guinea pigs as well as pigs; birds, including domestic, wild and game birds such as chickens, turkeys and other gallinaceous birds, ducks, geese, and the like. The terms do not denote a particular age. Thus, both adult and newborn individuals are intended to be covered. The methods described herein are intended for use in any of the above vertebrate species, since the immune systems of all of these vertebrates operate similarly.

In some instances, the invention may be administered to any suitable subject and in particular any suitable subject of a given species, preferably a suitable human subject. Thus, as many subjects as possible may, for instance, be subject to administration without emphasis on any particular group of subjects. For instance, a population of subjects as a whole, or as many as possible, may be subject to administration.

The protein, particle or nucleic acid of the invention is for administration to a subject. It may be administered simultaneously or sequentially with an adjuvant. Therefore the composition of the invention comprising the protein, particle or nucleic acid may also comprise an adjuvant. The composition of the invention may be one which is to be delivered by injection (such as intradermal, subcutaneous, intramuscular, intravenous, intraosseous, and intraperitoneal), transdermal particle delivery, inhalation, topically, orally or transmucosally (such as nasal, sublingual, vaginal or rectal).

The compositions may be formulated as conventional pharmaceutical preparations. This can be done using standard pharmaceutical formulation chemistries and methodologies, which are available to those skilled in the art. For example, compositions containing the protein, particle or nucleic acid with or without an adjuvant can be combined with one or more pharmaceutically acceptable excipients or vehicles to provide a liquid preparation. Thus also provided is a pharmaceutical composition comprising the protein, particle or nucleic acid together with a pharmaceutically acceptable carrier or diluent. The composition optionally comprises an adjuvant.

Auxiliary substances, such as wetting or emulsifying agents, pH buffering substances and the like, may be present. These carriers, diluents and auxiliary substances are generally pharmaceutical agents which may be administered without undue toxicity and which, in the case of antigenic compositions will not in themselves induce an immune response in the individual receiving the composition. Pharmaceutically acceptable carriers include, but are not limited to, liquids such as water, saline, polyethyleneglycol, hyaluronic acid, glycerol and ethanol. Pharmaceutically acceptable salts can also be included therein, for example, mineral acid salts such as hydrochlorides, hydrobromides, phosphates, sulfates, and the like; and the salts of organic acids such as acetates, propionates, malonates, benzoates, and the like. It is also preferred, although not required, that the preparation will contain a pharmaceutically acceptable carrier that serves as a stabilizer, particularly for peptide, protein or other like molecules if they are to be included in the composition. Examples of suitable carriers that also act as stabilizers for peptides include, without limitation, pharmaceutical grades of dextrose, sucrose, lactose, trehalose, mannitol, sorbitol, inositol, dextran, and the like. Other suitable carriers include, again without limitation, starch, cellulose, sodium or calcium phosphates, citric acid, tartaric acid, glycine, high molecular weight polyethylene glycols (PEGs), and combination thereof. A thorough discussion of pharmaceutically acceptable excipients, vehicles and auxiliary substances is available in REMINGTON'S PHARMACEUTICAL SCIENCES (Mack Pub. Co., N.J. 1991).

Certain facilitators of nucleic acid uptake and/or expression ("transfection facilitating agents") can also be included in the compositions, for example, facilitators such as bupivacaine, cardiotoxin and sucrose, and transfection facilitating vehicles such as liposomal or lipid preparations that are routinely used to deliver nucleic acid molecules. Anionic and neutral liposomes are widely available and well known for delivering nucleic acid molecules (see, e.g., Liposomes: A Practical Approach, (1990) RPC New Ed., IRL Press). Cationic lipid preparations are also well known vehicles for use in delivery of nucleic acid molecules. Suitable lipid preparations include DOTMA (N-[1-(2,3-dioleyloxy)propyl]-N,N,N-trimethylammonium chloride), available under the tradename Lipofectin™ , and DOTAP (1,2-bis(oleyloxy)-3-(trimethylammonio)propane), see, e.g., Felgner et al. (1987) Proc. Natl. Acad. Sci. USA 84:7413-7416; Malone et al. (1989) Proc. Natl. Acad. Sci. USA 86:6077-6081; US Patent Nos 5,283,185 and 5,527,928, and International Publication Nos WO 90/11092, WO 91/15501 and WO 95/26356. These cationic lipids may preferably be used in association with a neutral lipid, for example DOPE (dioleyl phosphatidylethanolamine). Still further transfection-facilitating compositions that can be added to the above lipid or liposome preparations include spermine derivatives (see, e.g., International Publication No. WO 93/18759) and membrane-permeabilizing compounds such as GALA, Gramicidine S and cationic bile salts (see, e.g., International Publication No. WO 93/19768).

Alternatively, the protein, particle or nucleic acid and/or the adjuvant may be encapsulated, adsorbed to, or associated with, particulate carriers. Suitable particulate carriers include those derived from polymethyl methacrylate polymers, as well as PLG microparticles derived from poly(lactides) and poly(lactide-co-glycolides). See, e.g., Jeffery et al. (1993) Pharm. Res. 10:362-368. Other particulate systems and polymers can also be used, for example, polymers such as polylysine, polyarginine, polyornithine, spermine, spermidine, as well as conjugates of these molecules. For example, polynucleotides can be precipitated onto carriers in the presence of a polynucleotide condensing agent and a metal ion chelating agent. Preferred condensing agents include cationic polymers, in particular polyamines, and in particular a polyargine or a polylysine. In a preferred instance the polyamine is (Arg)4 or (Arg)6. Reference may be made to the techniques discussed in WO2004/208560 which may be employed.

Once formulated the compositions can be delivered to a subject *in vivo* using a variety of known routes and techniques. For example, the liquid preparations can be provided as an injectable solution, suspension or emulsion and administered via parenteral, subcutaneous, intradermal, intramuscular, intravenous intraosseous and intraperitoneal injection using a conventional needle and syringe, or using a liquid jet injection system. Liquid preparations can also be administered topically to skin or mucosal tissue (e.g. nasal, sublingual, vaginal or rectal), or provided as a finely divided spray suitable for respiratory or pulmonary administration. Other modes of administration include oral administration, suppositories, and active or passive transdermal delivery techniques.

The protein, particle or nucleic acid of the invention is administered to a subject in an amount that will be effective in treating a disease or condition or in modulating an immune response. An appropriate effective amount will fall in a relatively broad range but can be readily determined by one of skill in the art by routine trials. The "Physicians Desk Reference" and "Goodman and Gilman's The Pharmacological Basis of Therapeutics" are useful for the purpose of determining the amount needed. Typically, the protein or particles are administered in a dose of from 0.1 to 200 mg, preferably from 1 to 100 mg, more preferably from 10 to 50 mg body weight. The nucleic acid of the invention may be administered directly as a naked nucleic acid construct using techniques known in the art or using vectors known in the art. The amount of nucleic acid administered is typically in the range of from 1 µg to 10 mg, preferably from 100 mg to 1 mg. The composition may be given in a single dose schedule or a multiple dose schedule, for example in from 2 to 32 or from 4 to 16 doses. The routes of administration and doses given above are intended only as a guide, and the route and dose may ultimately be at the discretion of the physician.

In some cases after an initial administration a subsequent administration of the composition of the invention may be performed. In particular, following an initial administration a subject may be given a "booster". The booster may be, for instance, a dose chosen from any of those mentioned herein. The booster administration may, for instance, be at least a week, two weeks, four weeks, six weeks, a month, two months or six months after the initial administration.

The protein, particle or nucleic acid of the invention and an adjuvant may be administered sequentially or simultaneously, preferably simultaneously. The two entities may be administered in the same or different compositions, preferably the same composition. An adjuvant is delivered so that an adjuvant effect is seen, that is the therapeutic effect or the immune response seen will differ from that if the adjuvant had not been administered with the antigen. The two entities may be administered at the same or different sites, preferably the same sites. Preferably, the two entities are administered in the same composition at the same site at the same time preferably via injection.

Any suitable adjuvant may be used. Currently used vaccine adjuvants include:
- Inorganic compounds, such as aluminium salts (e.g. aluminium hydroxide and aluminium phosphate) or calcium phosphate. Aluminium salts are otherwise known as alum.
- Oil emulsions and surfactant based formulations, e.g. MF59 (microfluidised detergent stabilised oil-in-water emulsion), QS21 (purified saponin), AS02 [SBAS2] (oil-in-water emulsion + MPL + QS-21), Montanide ISA-51 and ISA-720 (stabilised water-in-oil emulsion).
- Particulate adjuvants, e.g. virosomes (unilamellar liposomal vehicles incorporating e.g. influenza haemagglutinin), AS04 ([SBAS4] Al salt with MPL), ISCOMS (structured complex of saponins and lipids), and polylactide co-glycolide (PLG).
- Microbial derivatives (natural and synthetic), e.g. monophosphoryl lipid A (MPL), Detox (MPL + *M. Phlei* cell wall skeleton), AGP [RC-529] (synthetic acylated monosaccharide), DC_Chol (lipoidal immunostimulators able to self organise into liposomes), OM-174 (lipid A derivative), CpG motifs (synthetic oligonucleotides containing immunostimulatory CpG motifs), and modified LT and CT (genetically modified bacterial toxins to provide non-toxic adjuvant effects).
- Endogenous human immunomodulators, e.g. hGM-CSF or hIL-12 (cytokines that can be administered either as protein or plasmid encoded), and Immudaptin (C3d tandem array).
- Inert vehicles, such as gold particles.

Preferably the adjuvant used is alum. Most preferably the adjuvant is a mixture of aluminium hydroxide and magnesium hydroxide, for example Inject alum (Pierce Laboratories).

### Diagnostic Tool

The protein of the invention may by useful as a diagnostic reagent, for example in detecting a disease, a pathogen or toxin. Thus the invention provides the use of a protein of the invention or a particle comprising multiple copies of a protein of the invention, for detecting an antigen, for example in a sample. The invention also provides a method of detecting an antigen in a sample, comprising applying a protein of the invention or a particle comprising multiple copies of a protein of the invention to the sample.

As discussed above, the flexibility of the technology means that the protein of the invention, in addition to comprising an sdAb, can comprise any other useful heterologous protein. Thus, when the protein of the invention is for use as a diagnostic tool, it may comprise a heterologous protein, for example a heterologous protein that provides a means of visualising the protein of the invention. This could be useful, for example, for tracking the location of the protein of the invention *in vivo* or for determining whether a sample contains the protein of the invention. Examples of such heterologous proteins include fluorescent or bioluminescent proteins, such GFP.

The invention is illustrated by the following Example:

### Example

Tandem HBcAg protein display technology was adapted to present a Camelid single-domain antibody (VHH) on the surface of HBcAg core-like particles. The anti-GFP "Enhancer" VHH was isolated from an immunized alpaca-derived VHH phage display library and thoroughly characterised by Kirchhofer et al (2010), who determined its affinity for GFP to be in the sub-nanomolar range. The gene coding for this VHH was cloned into the C-terminal monomer of the tandem core construct. This construct, named tHB-VHH, was expressed transiently in *Nicotiana benthamiana* and directed the production of core-like particles (CLPs) displaying the VHH on the surface (Figure 4).

Particles were estimated to be produced in the range of hundreds of milligrams per kilogram of fresh weight tissue, and the particles were shown to cause GFP to migrate down a sucrose gradient, which it does not normally do as it is not dense enough on its own or in the presence of non-VHH bearing tHB particles (Figure 5). This clearly shows that the tHB-VHH particles bind GFP, thus indicating that the VHH is properly folded and active on the surface of the CLPs. Western blot analysis of the sucrose cushion fractions confirmed that GFP remains largely in the supernatant when run on its own or with tHB, but when run with tHB-VHH, GFP and CLPs co-localise at the bottom of the cushion (Figure 6).

It is worth noting that antibody-display on the surface of tandem cores in plants was also attempted with the more commonly used single-chain variable fragment antibodies (scFv), but expression yields were very low and no particle formation was observed (data not shown). This is most likely due to the more complicated structure of scFv, which have two domains corresponding to the variable regions of the light and heavy chains, whereas VHH only have a single domain corresponding to the variable region of the heavy chain. This increased complexity of scFv probably prevented proper folding of the insert when both N- and C-termini were attached to the e1 loop of HBcAg, leading to improper folding of the entire tandem molecule.

It is also interesting to note that VHH could not be displayed on the surface of monomeric (i.e. non-tandem) HBcAg particles in plants: the recombinant protein could not be detected (Figure 7), indicating that folding problems or steric hindrance issues probably caused immediate degradation of the protein.

### Methods

The amino acid sequence of the anti-GFP VHH was obtained from GenBank protein database (accession 3K1K_C) and the gene was synthesised by GeneArt (Life Technologies) with codon usage optimised for expression in *N. benthamiana.* The VHH gene was then cloned in-frame into the e1 loop of the C-terminal monomer of the tandem core construct, with a (GlyGlyGlyGlySer)₃ linker on each end of the VHH sequence. The tandem core construct containing the VHH gene is called tHBcAg-VHH.

The tHBcAg-VHH construct was expressed in 3-week old *N. benthamiana* plants using the pEAQ-HT vector (Sainsbury, Thuenemann et al. 2009), the use of which has been reviewed in Peyret and Lomonossoff (2013). After seven days post-infiltration (7 dpi), the infiltrated leaf tissue was harvested and blended in three volumes of 0.1 M sodium phosphate buffer, pH 7, supplemented with cOmplete EDTA-free protease inhibitor cocktail tablets (Roche). The crude extracts were filtered over Miracloth (Calbiochem) and the filtrates were clarified by centrifugation at 9,000 g for 10 min at 4°C. The clarified extracts were then underlain with 2 ml 25%, then 0.5 ml 70%, sucrose solutions in UltraClear 14X89 mm ultracentrifuge tunes (Beckman Coulter) and centrifuged at 273,800 g in a TH641 ultracentrifuge swing-out rotor (Sorvall) for 2 h 30 min at 4°C (results shown in Figure 5).

For western blotting (Figure 6), the sucrose gradients were fractionated from the bottom into four fractions (bottom 0.5 µl, middle 0.5 µl, and top 1.5 µl of the sucrose cushion, and crude extract supernatant). Duplicate 4-12% gradient NuPage (Life Technologies) SDS-PAGE gels were loaded with 5 µl denatured sample for each fraction (1.25 µl of each fraction boiled in 3.75 µl of 1:3 LDS: β-mercaptoethanol for 25 minutes). The proteins were transferred to Amersham Hybond-ECL nitrocellulose membranes (GE Healthcare), and blotted with either anti-HBcAg monoclonal primary antibody (clone 10E11, Abcam) with goat anti-mouse horseradish peroxidase - conjugated secondary antibody (Invitrogen); or with anti-GFP horseradish peroxidase - conjugated antibody (Life Technologies). The chemiluminescent signal was detected on Amersham Hyperfilm ECL (GE Healthcare).

For electron microscopy (Figure 4), particles were purified over successive sucrose cushions before being dialysed against ammonium bicarbonate and concentrated on a Speed Vac (Savant). Concentrated particles were then imaged on a Tecnai 20 electron miscroscope (FEI) by staining with uranyl acetate on a 400 mesh copper/palladium grid.

The tests for HB-VHH expression were carried out on 6 dpi plants expressing either an empty vector pEAQ-HT control, tHB-VHH, or monomeric HB-VHH from one of three different *Agrobacterium* clones. Leaves were harvested and 6 leaf discs (90 mg) were cut out of infiltrated areas of the leaves with a cork borer and these were supplemented with 270 µl extraction buffer and a ceramic bead. Leaf discs were disrupted on the Omni Bead Ruptor 24 homogenizer (Camlab) at speed 4 for 30 seconds. The samples were then centrifuged at 16,000 g for 10 minutes and 100 µl of the supernatants were mixed with 50 µl of 3:1 LDS: β-mercaptoethanol then boiled for 30 minutes, and 20 µl of each denatured sample were run on the gel. The western blot was carried out as above, with the anti-HBcAg antibody (Figure 7).

### References

Eyer, L. and K. Hruska (2012). "Single-domain antibody fragments derived from heavy-chain antibodies: a review". Vet. Medicina 57(9):439-513.
Kirchhofer, A., J. Helma, et al. (2010). "Modulation of protein properties in living cells using nanobodies." Nat Struct Mol Biol 17(1): 6.
Peyret, H. and G. Lomonossoff (2013). "The pEAQ vector series: the easy and quick way to produce recombinant proteins in plants." Plant Molecular Biology: 1-8.
Sainsbury, F., E. C. Thuenemann, et al. (2009). "pEAQ: versatile expression vectors for easy and quick transient expression of heterologous proteins in plants." Plant Biotechnology Journal 7(7): 682-693.

## Claims

1. A protein comprising a first and a second copy of hepatitis B core antigen (HBcAg) in tandem, in which one or both of the copies of HBcAg comprises a single-domain antibody in the e1 loop.

2. The protein according to claim 1, wherein:
- both copies of HBcAg comprise a single-domain antibody in the e1 loop; or
- the first copy of HBcAg comprises said single-domain antibody in the e1 loop and the second copy of HBcAg comprises a heterologous protein in the e1 loop, optionally wherein the heterologous protein comprises an epitope or is a fluorescent or bioluminescent protein.

3. The protein according to any one of the preceding claims, wherein the single-domain antibody is bound to an antigen.

4. The protein according to any one of the preceding claims, wherein:
(a) the tandem copies of HBcAg are joined by a linker; and/or
(b) the single-domain antibody in the e1 loop of one or both copies of the HBcAg is flanked on one or both sides by a linker.

5. The protein according to claim 4, wherein:
(a) the or each linker is at least 1.5 nm in length; and/or
(b) the or each linker comprises multiple copies of the sequence GlyₙSer(GₙS) wherein n is from 2 to 8.

6. A particle comprising multiple copies of a protein as claimed in any one of the preceding claims.

7. A nucleic acid molecule encoding a protein as claimed in any one of claims 1 to 5, optionally wherein the nucleic acid molecule is an expression vector.

8. A host cell comprising a nucleic acid molecule as claimed in claim 7.

9. A process for producing a protein as claimed in any one of claims 1 to 5, which process comprises culturing a host cell containing a nucleic acid molecule which encodes the protein under conditions in which the protein is expressed, and recovering the protein.

10. A pharmaceutical composition comprising a protein as claimed in any one of claims 1 to 5, a particle as claimed in claim 6 or a nucleic acid molecule as claimed in claim 7, and a pharmaceutically acceptable carrier or diluent, optionally further comprising an adjuvant.

11. A vaccine comprising a protein as claimed in any one of claims 1 to 5, a particle as claimed in claim 6 or a nucleic acid molecule as claimed in claim 7, and a pharmaceutically acceptable carrier or diluent, optionally further comprising an adjuvant.

12. A protein according to any one of claims 1 to 5 or a particle according to claim 6, for use in a method of treatment of the human or animal body.

13. A protein according to any one of claims 1 to 5 or a particle according to claim 6, for use in a method of vaccination of the human or animal body.

14. Use of a protein according to claim 1 or 2 or a particle comprising multiple copies of a protein according to claim 1 or 2, for detecting an antigen in a sample.

15. A method of detecting an antigen in a sample, comprising applying a protein according to claim 1 or 2 or a particle comprising multiple copies of a protein according to claim 1 or 2 to the sample.

## Patentansprüche

1. Protein, das eine erste und eine zweite Kopie eines Hepatitis-B-Kernantigens (HBcAg) in Tandem umfasst, in dem eine oder beide Kopien des HBcAg einen Einzeldomänenantikörper in der e1-Schleife umfasst.

2. Protein nach Anspruch 1, wobei:
- beide Kopien des HBcAg einen Einzeldomänenantikörper in der e1-Schleife umfassen; oder
- die erste Kopie des HBcAg den Einzeldomänenantikörper in der e1-Schleife umfasst und die zweite Kopie des HBcAg ein heterologes Protein in der e1-Schleife umfasst, optional wobei das heterologe Protein ein Epitop umfasst oder ein fluoreszierendes oder biolumineszierendes Protein ist.

3. Protein nach einem der vorhergehenden Ansprüche, wobei der Einzeldomänenantikörper an ein Antigen gebunden ist.

4. Protein nach einem der vorhergehenden Ansprüche, wobei:
(a) die Tandemkopien des HBcAg durch einen Linker verbunden sind; und/oder
(b) der Einzeldomänenantikörper in der e1-Schleife von einer oder beiden Kopien des HBcAg an einer oder beiden Seiten durch einen Linker flankiert ist.

5. Protein nach Anspruch 4, wobei:
(a) der oder jeder Linker mindestens 1,5 nm lang ist; und/oder
(b) der oder jeder Linker mehrere Kopien der Sequenz GlyₙSer(GₙS) umfasst, wobei n von 2 bis 8 ist.

6. Partikel, umfassend mehrere Kopien eines Proteins nach einem der vorhergehenden Ansprüche.

7. Nukleinsäuremolekül, das ein Protein kodiert, nach einem der Ansprüche 1 bis 5, wobei das Nukleinsäuremolekül optional ein Expressionsvektor ist.

8. Wirtszelle, umfassend ein Nukleinsäuremolekül nach Anspruch 7.

9. Verfahren zum Herstellen eines Proteins nach einem der Ansprüche 1 bis 5, wobei das Verfahren ein Kultivieren einer Wirtszelle, die ein Nukleinsäuremolekül enthält, das das Protein unter Bedingungen kodiert, bei denen das Protein exprimiert wird, und ein Gewinnen des Proteins umfasst.

10. Pharmazeutische Zusammensetzung, umfassend ein Protein nach einem der Ansprüche 1 bis 5, ein Partikel nach Anspruch 6 oder ein Nukleinsäuremolekül nach Anspruch 7 und einen pharmazeutisch annehmbaren Träger oder Verdünner, optional ferner umfassend ein Adjuvans.

11. Impfstoff, umfassend ein Protein nach einem der Ansprüche 1 bis 5, ein Partikel nach Anspruch 6 oder ein Nukleinsäuremolekül nach Anspruch 7 und einen pharmazeutisch annehmbaren Träger oder Verdünner, optional ferner umfassend ein Adjuvans.

12. Protein nach einem der Ansprüche 1 bis 5 oder Partikel nach Anspruch 6 zur Verwendung in einem Verfahren des Behandelns des menschlichen oder tierischen Körpers.

13. Protein nach einem der Ansprüche 1 bis 5 oder Partikel nach Anspruch 6 zur Verwendung in einem Verfahren der Impfung des menschlichen oder tierischen Körpers.

14. Verwendung eines Proteins nach Anspruch 1 oder 2 oder eines Partikels, das mehrere Kopien eines Proteins nach Anspruch 1 oder 2 umfasst, um ein Antigen in einer Probe nachzuweisen.

15. Verfahren des Nachweisens eines Antigens in einer Probe, umfassend ein Aufbringen eines Proteins nach Anspruch 1 oder 2 oder eines Partikels, das mehrere Kopien eines Proteins nach Anspruch 1 oder 2 umfasst, auf die Probe.

## Revendications

1. Protéine comprenant des première et deuxième copies d'antigène nucléocapsidique de l'hépatite B (HBcAg) en tandem, dans laquelle l'une des copies ou les deux copies de HBcAg comprennent un anticorps à domaine unique dans la boucle e1.

2. Protéine selon la revendication 1, dans laquelle :
- les deux copies de HBcAg comprennent un anticorps à domaine unique dans la boucle e1 ; ou
- la première copie de HBcAg comprend un anticorps à domaine unique dans la boucle e1 et la deuxième copie de HBcAg comprend une protéine hétérologue dans la boucle e1, la protéine hétérologue comprenant éventuellement un épitope ou étant une protéine fluorescente ou bioluminescente.

3. Protéine selon l'une quelconque des revendications précédentes, dans laquelle l'anticorps à domaine unique est lié à un antigène.

4. Protéine selon l'une quelconque des revendications précédentes, dans laquelle :
(a) les copies de HBcAg en tandem sont jointes par un lieur ; et/ou
(b) l'anticorps à domaine unique dans la boucle e1 ou l'une des copies ou les deux copies de HBcAg sont accompagnés sur un côté ou les deux côtés par un lieur.

5. Protéine selon la revendication 4, dans laquelle :
(a) le ou chaque lieur a une longueur d'au moins 1,5 nm ; et/ou
(b) le ou chaque lieur comprend plusieurs copies de la séquence GlyₙSer(GₙS) où n vaut 2 à 8.

6. Particule comprenant plusieurs copies d'une protéine telle que revendiquée dans l'une quelconque des revendications précédentes.

7. Molécule d'acide nucléique codant pour une protéine telle que revendiquée dans l'une quelconque des revendications 1 à 5, la molécule d'acide nucléique étant éventuellement un vecteur d'expression.

8. Cellule hôte comprenant une molécule d'acide nucléique telle que revendiquée dans la revendication 7.

9. Processus de production d'une protéine telle que revendiquée dans l'une quelconque des revendications 1 à 5, le processus comprenant la mise en culture d'une cellule hôte contenant une molécule d'acide nucléique qui code pour la protéine dans des conditions dans lesquelles la protéine est exprimée, et le recueil de la protéine.

10. Composition pharmaceutique comprenant une protéine telle que revendiquée dans l'une quelconque des revendications 1 à 5, une particule telle que revendiquée dans la revendication 6 ou une molécule d'acide nucléique telle que revendiquée dans la revendication 7 et un support ou diluant pharmaceutiquement acceptable, comprenant éventuellement en outre un adjuvant.

11. Vaccin comprenant une protéine telle que revendiquée dans l'une quelconque des revendications 1 à 5, une particule telle que revendiquée dans la revendication 6 ou une molécule d'acide nucléique telle que revendiquée dans la revendication 7 et un support ou diluant pharmaceutiquement acceptable, comprenant éventuellement en outre un adjuvant.

12. Protéine selon l'une quelconque des revendications 1 à 5 ou particule selon la revendication 6, destinée à une utilisation dans un procédé de traitement du corps humain ou animal.

13. Protéine selon l'une quelconque des revendications 1 à 5 ou particule selon la revendication 6, destinée à une utilisation dans un procédé de vaccination du corps humain ou animal.

14. Utilisation d'une protéine selon la revendication 1 ou 2 ou d'une particule comprenant plusieurs copies d'une protéine selon la revendication 1 ou 2, pour détecter un antigène dans un échantillon.

15. Procédé de détection d'un antigène dans un échantillon, comprenant l'application à l'échantillon d'une protéine selon la revendication 1 ou 2 ou d'une particule comprenant plusieurs copies d'une protéine selon la revendication 1 ou 2.


### SEQUENCE LISTING

<110> iQur Limited
<120> ANTIBODY DISPLAY
<130> N400310US
<160> 2
<170> PatentIn version 3.5
<210> 1
   <211> 639
   <212> DNA
   <213> Hepatitis B virus
<220>
   <221> CDS
   <222> (1)..(639)
<400> 1
   atg caa ctt ttt cac ctc tgc cta atc atc tct tgt tca tgt cct act        48
   Met Gln Leu Phe His Leu Cys Leu Ile Ile Ser Cys Ser Cys Pro Thr
   1               5                   10                  15
   gtt caa gcc tcc aag ctg tgc ctt ggg tgg ctt tgg ggc atg gac atc        96
   Val Gln Ala Ser Lys Leu Cys Leu Gly Trp Leu Trp Gly Met Asp Ile
               20                  25                  30
   gac cct tat aaa gaa ttt gga gct act gtg gag tta ctc tcg ttt ttg       144
   Asp Pro Tyr Lys Glu Phe Gly Ala Thr Val Glu Leu Leu Ser Phe Leu
           35                  40                  45
   cct tct gac ttc ttt cct tca gta cga gat ctt cta gat acc gcc tca       192
   Pro Ser Asp Phe Phe Pro Ser Val Arg Asp Leu Leu Asp Thr Ala Ser
       50                  55                  60
   gct ctg tat cgg gaa gcc tta gag tct cct gag cat tgt tca cct cac       240
   Ala Leu Tyr Arg Glu Ala Leu Glu Ser Pro Glu His Cys Ser Pro His
   65                  70                  75                  80
   cat act gca ctc agg caa gca att ctt tgc tgg ggg gaa cta atg act       288
   His Thr Ala Leu Arg Gln Ala Ile Leu Cys Trp Gly Glu Leu Met Thr
                   85                  90                  95
   cta gct acc tgg gtg ggt gtt aat ttg gaa gat cca gcg tct aga gac       336
   Leu Ala Thr Trp Val Gly Val Asn Leu Glu Asp Pro Ala Ser Arg Asp
               100                 105                 110
   cta gta gtc agt tat gtc aac act aat atg ggc cta aag ttc agg caa       384
   Leu Val Val Ser Tyr Val Asn Thr Asn Met Gly Leu Lys Phe Arg Gln
           115                 120                 125
   ctc ttg tgg ttt cac att tct tgt ctc act ttt gga aga gaa aca gtt       432
   Leu Leu Trp Phe His Ile Ser Cys Leu Thr Phe Gly Arg Glu Thr Val
       130                 135                 140
   ata gag tat ttg gtg tct ttc gga gtg tgg att cgc act cct cca gct       480
   Ile Glu Tyr Leu Val Ser Phe Gly Val Trp Ile Arg Thr Pro Pro Ala
   145                 150                 155                 160
   tat aga cca cca aat gcc cct atc cta tca aca ctt ccg gag act act        528
   Tyr Arg Pro Pro Asn Ala Pro Ile Leu Ser Thr Leu Pro Glu Thr Thr
                   165                 170                 175
   gtt gtt aga cga cga ggc agg tcc cct aga aga aga act ccc tcg cct        576
   Val Val Arg Arg Arg Gly Arg Ser Pro Arg Arg Arg Thr Pro Ser Pro
               180                 185                 190
   cgc aga cga agg tct caa tcg ccg cgt cgc aga aga tct caa tct cgg        624
   Arg Arg Arg Arg Ser Gln Ser Pro Arg Arg Arg Arg Ser Gln Ser Arg
           195                 200                 205
   gaa tct caa tgt tag                                                    639
   Glu Ser Gln Cys
       210
<210> 2
   <211> 212
   <212> PRT
   <213> Hepatitis B virus
<400> 2
   Met Gln Leu Phe His Leu Cys Leu Ile Ile Ser Cys Ser Cys Pro Thr
   1               5                   10                  15
   Val Gln Ala Ser Lys Leu Cys Leu Gly Trp Leu Trp Gly Met Asp Ile
               20                  25                  30
   Asp Pro Tyr Lys Glu Phe Gly Ala Thr Val Glu Leu Leu Ser Phe Leu
           35                  40                  45
   Pro Ser Asp Phe Phe Pro Ser Val Arg Asp Leu Leu Asp Thr Ala Ser
       50                  55                  60
   Ala Leu Tyr Arg Glu Ala Leu Glu Ser Pro Glu His Cys Ser Pro His
   65                  70                  75                  80
   His Thr Ala Leu Arg Gln Ala Ile Leu Cys Trp Gly Glu Leu Met Thr
                   85                  90                  95
   Leu Ala Thr Trp Val Gly Val Asn Leu Glu Asp Pro Ala Ser Arg Asp
               100                 105                 110
   Leu Val Val Ser Tyr Val Asn Thr Asn Met Gly Leu Lys Phe Arg Gln
           115                 120                 125
   Leu Leu Trp Phe His Ile Ser Cys Leu Thr Phe Gly Arg Glu Thr Val
       130                 135                 140
   Ile Glu Tyr Leu Val Ser Phe Gly Val Trp Ile Arg Thr Pro Pro Ala
   145                 150                 155                 160
   Tyr Arg Pro Pro Asn Ala Pro Ile Leu Ser Thr Leu Pro Glu Thr Thr
                   165                 170                 175
   Val Val Arg Arg Arg Gly Arg Ser Pro Arg Arg Arg Thr Pro Ser Pro
               180                 185                 190
   Arg Arg Arg Arg Ser Gln Ser Pro Arg Arg Arg Arg Ser Gln Ser Arg
           195                 200                 205
   Glu Ser Gln Cys
       210